# EUROPÄISCHE PATENTSCHRIFT

(11) **EP 0 543 247 B1**
(45) Veröffentlichungstag und Bekanntmachung des Hinweises auf die Patenterteilung: **13.09.1995**
(21) Anmeldenummer: 92119130.0
(22) Anmeldetag: 09.11.1992
(51) Int. Cl.: C01B 33/20, B01J 29/04, B01J 20/10

(54) **Verfahren zur Herstellung von im wesentlichen alkalifreien Titansilikat-Kristallen mit Zeolithstruktur**
Method for preparing titanium silicate crystals of zeolite structure and being essentially free of alkali metals
Procédé de préparation de cristaux de silicate de titane à structure zéolite essentiellement exempts de métaux alcalins

(30) Priorität: 21.11.1991 DE 4138155
(43) Veröffentlichungstag der Anmeldung: 26.05.1993
(73) Patentinhaber: BASF Aktiengesellschaft, 67063 Ludwigshafen (DE)
(72) Erfinder: Mueller, Ulrich, Dr., W-6730 Neustadt (DE); Hoelderich, Wolfgang, Prof. Dr., W-6710 Frankenthal (DE)

(56) Entgegenhaltungen:
- EP-A- 0 376 453
- WO-A-85/04853
- FR-A- 2 471 950

## Beschreibung

Die vorliegende Erfindung betrifft ein Verfahren zur Herstellung von im Wesentlichen alkalifreien Titansilikat-Kristallen mit Zeolithstruktur in Abwesenheit eines Metallhydroxids oder eines Metallsalzes unter hydrothermalen Bedingungen.

Zeolithe sind bekanntermaßen kristalline Alumosilikate mit geordneten Kanal- und Käfigstrukturen, deren Porenöffnungen im Bereich von Mikroporen kleiner 0,9 nm liegen. Das Netzwerk solcher Zeolithe ist aufgebaut aus SiO₄- und AlO₄-Tetraedern, die über gemeinsame Sauerstoffbrücken verbunden sind. Eine Übersicht der bekannten Strukturen findet sich beispielsweise bei W.M. Meier und D.H. Olson, "Atlas of Zeolite Structure Types", 2. Auflage, Butterworths, London 1987.

Zum Ausgleich der negativen Elektrovalenz, die durch den Einbau von Al(III) in das Si(IV)-Silikatgitter entsteht, findet man bei Zeolithen austauschfähige Kationen, insbesondere kann es sich dabei je nach Herstellverfahren um Kationen des Natriums, Kaliums, Lithiums oder Cäsiums handeln. Ersetzt man diese Kationen gegen Protonen, beispielsweise durch einen Ionenaustausch, so erhält man die entsprechend aziden Festkörper mit Zeolithstruktur, die sogenannte H-Form.

Aus der US-A-3,329,481 sind Materialien bekannt, bei denen im Silikatgitter anstelle des Si(IV) Titan als Ti(IV) sein soll. Diese Titanzeolithe, insbesondere solche mit einer Kristallstruktur vom MFI-Typ (vgl. Meier, Olson, a.a.O), sowie Möglichkeiten zu ihrer Herstellung sind beschrieben beispielsweise in der US-A-4,410,501, EP-A-311 983, US-A-4,666,692, DE-A-30 47 798 oder in BE-A-10 01 038. Titanhaltige Zeolithe mit anderen Strukturen kennt man aus EP-A-405 978. Außer Silizium und Titan können solche Materialien auch zusätzliche Elemente wie Aluminium (DE-A-31 41 283); Gallium (EP-A-266 825); Bor (US-A-4,666,692) oder geringe Mengen an Fluor enthalten (EP-A-292 363).

Titanzeolithe mit MFI-Struktur sind dafür bekannt, daß sie über ein bestimmtes Muster bei der Bestimmung ihrer Röntgenbeugungsaufnahmen, sowie zusätzlich über eine Gerüstschwingungsbande im IR-Bereich bei etwa 960 cm⁻¹ identifiziert werden können (DE-A-30 47 798) und sich damit von Alkalititanaten oder kristallinen und amorphen TiO₂-Phasen unterscheiden.

Von Titanzeolithen mit MFI-Struktur ist bekannt, daß sie sich als Katalysatoren für Oxidationsreaktionen eignen (B. Notari, Stud. Surf. Sci. Catal., Vol. 37, Amsterdam S. 413 bis 425 (1987)). So ist beispielsweise aus EP-A-100 118 und EP-A-100 119 ein Verfahren bekannt, wonach Propen in wäßriger Phase mit Wasserstoffperoxid an Titanzeolithen zu Propylenoxid epoxidiert werden kann. Die Herstellung von Cyclohexanonoxim aus Cyclohexanon durch Umsetzung mit Ammoniak und Wasserstoffperoxid lehrt EP-A-208 311 und die Hydroxylierung von Aromaten kennt man aus GB-A-21 16 974. Die Oxidation gesättigter Kohlenwasserstoffe von C₂ bis C₁₈ mit H₂O₂ an o.g. Titanzeolithen beschreibt EP-A-376 453.

Typischerweise stellt man die vorgenannten Titanzeolithe dadurch her, daß man eine wäßrige Mischung aus einer SiO₂-Quelle, einem Titandioxid und einer stickstoffhaltigen organischen Base, wie etwa Tetrapropylammoniumhydroxid, gegebenenfalls noch unter Hinzufügen von Alkali in einem Druckbehälter unter erhöhter Temperatur im Zeitraum mehrerer Stunden oder weniger Tage umsetzt, wobei das kristalline Produkt entsteht. Dieses wird abfiltriert, gewaschen, getrocknet und zur Entfernung der organischen Stickstoffbase bei erhöhter Temperatur gebrannt. In dem so erhaltenen Pulver liegt das Titan zumindest teilweise innerhalb des Zeolithgerüsts in wechselnden Anteilen mit vier-, fünf- oder sechsfacher Koordination vor (Behrens, et al.; J. Chem. Soc., Chem. Commun. 1991, S. 678 bis 680). Zur Verbesserung des katalytischen Verhaltens kann sich noch eine mehrmalige Waschbehandlung mit schwefelsaurer Wasserstoffperoxidlösung anschließen, worauf das Titanzeolith-Pulver erneut getrocknet und gebrannt werden muß, wie es etwa in EP-A-267 362 beschrieben wird. Der pulverförmige Titanzeolith muß nun abschließend in Zusätzen mit einem geeigneten inerten Binder in einem Formgebungsschritt verarbeitet werden, um ihn als Katalysator in einer handhabbaren Form verfügbar zu machen. Eine Methode hierzu wird in EP-A-200 260 aufgezeigt. Aus WO-A-85/04853 ist ein Verfahren zur Herstellung von Titansilikat-Kristallen mit Zeolithstruktur bekannt, bei dem man eine SiO₂-haltige Mischung aus Wasser, einem Tetraalkylamoniumhydroxid, einer Titankomponente und Ammoniak in Abwesenheit eines Metallhydroxids oder eines Metallsalzes hydrothermal umsetzt.

Die bislang bekannten Verfahren zur Herstellung von Titanzeolithen mit MFI-Struktur haben einige schwerwiegende Nachteile.

So ist etwa Tetrapropylammoniumhydroxid TPAOH, mit geringem Restgehalt an Alkali, insbesondere Kalium, ein sehr teurer Einsatzstoff und muß in hohen Konzentrationen eingesetzt werden. Die Verarbeitung der mittels TPAOH hergestellten Titanzeolithpulver in Formgebungsschritten, zur Fertigung von handhabbaren Katalysatoren, ist arbeitsaufwendig und erfordert nach einer Verstrangung mit oder ohne inerten Binder einen weiteren energieintensiven und zeitaufwendigen Trocknungs- und Kalzinierschritt.

Der vorliegenden Erfindung lag daher die Aufgabe zugrunde, den zuvor vorgenannten Nachteilen abzuhelfen.

Demgemäß wurde ein neues und verbessertes Verfahren zur Herstellung von im Wesentlichen alkalifreien Titansilikat-Kristallen mit Zeolithstruktur gefunden, welches dadurch gekennzeichnet ist, daß man eine SiO₂-haltige Mischung aus Wasser, gelöstem Tetra-n-propylammoniumhalogenid mit einem molaren Verhältnis von Tetrapropylammoniumhalogenid/SiO₂ im Bereich 0,05:1 bis 0,015:1, einer Titankomponenten und Ammoniak in Abwesenheit eines Metallhydroxids oder eines Metallsalzes bei einem molaren Verhältnis von Ammoniak/Tetraalkylammonium von 3:1 bis 200:1 hydrothermal umsetzt.

Das erfindungsgemäße Verfahren bietet die Möglichkeit die Kristallisation von Titansilikaten mit Zeolithstruktur durch hydrothermale Umsetzung einer SiO₂-Quelle mit einer Titankomponente in Gegenwart wäßriger Lösungen von schwach konzentriertem Tetrapropylammoniumhalogeniden und Ammoniak. Dabei ermöglicht es das erfindungsgemäße Verfahren, daß man die entstehenden Titanzeolith-Kristalle in hoher Ausbeute direkt in Form plättchenförmiger, weitestgehend alkalifreier, großer Primärkristallite erhält, die aufgrund ihrer Teilchengröße ohne weitere Verformungsschritte als Katalysatoren zur Umsetzung organischer Moleküle und zwar insbesonin Wirbelschicht-, Rieselbett- oder Suspensionsfahrweisen verwendet werden können.

Als Tetrapropylammoniumhalogenide (TPAH) eignen sich beispielsweise Tetrapropylammoniumfluorid, Tetrapropylammoniumchlorid, Tetrapropylammoniumbromid, bevorzugt Tetrapropylammoniumbromid. Das molares Verhältnis von TPAH/SiO₂ beträgt von 0,042 : 1 bis 0,2 : 1, vorzugsweise von 0,042 : 1 bis 0,15 : 1. Als vorteilhaft hat es sich gezeigt, daß man die Kristallisation der Titansilikate mit Zeolithstruktur hydrothermal bei Temperaturen von 100 bis 250°C, besonders bei 120 bis 200°C und insbesondere bei 150 bis 190°C durchführt. Dabei ist es angebracht, daß man dabei ein molares Verhältnis von, als wäßrige Lösung eingesetztem Ammoniak, zu SiO₂ von 10 : 1 bis 1 : 1, bevorzugt von 6 : 1 bis 1,5 : 1 und besonders bevorzugt von 5 : 1 bis 3 : 1 einhält.

Nach dem erfindungsgemäßen Verfahren erhält man katalytisch verwendbare Titansilikat-Zeolithe dadurch, daß man die Titankomponente in Form einer löslichen, wäßrigen oder wäßrig-/alkoholischen Peroxotitanatverbindung in der vorbeschriebenen Weise bei der hydrothermalen Umsetzung der Reaktionsmischung zusetzt. Dies gelingt dadurch, daß man SiO₂/TiO₂ im Molverhältnis von 10:1 bis 1500:1, bevorzugt von 10:1 bis 250:1, besonders bevorzugt von 10 bis 100 einsetzt und/oder bei einer Verdünnung von SiO₂/H₂O mit 0,07:1 bis 0,025:1, bevorzugt von 0,05:1 bis 0,04:1 arbeitet.

Das erfindungsgemäße alkalifreie Verfahren macht es möglich, daß darüber hinaus das Material nach einer Temperaturbehandlung bei 350 bis 600°C, bevorzugt bei 400 bis 550°C und besonders bevorzugt bei 450 bis 500°C direkt und ohne zusätzlichen Ionenaustausch und insbesondere aufgrund der Kristallgröße von 1 bis 50 µm, bevorzugt 3 bis 30 µm und der plättchenförmigen Kristallform ohne weitere Formgebung in einer katalytisch wirksamen Form vorliegt und als Katalysator verwendet werden kann.

Die erfindungsgemäß hergestellten, aminfreien Titansilikate mit MFI-Struktur verfügen in ihrem IR-Spektrum über eine charakteristische Bande bei 950 bis 960 cm⁻¹, wohingegen die noch das Tetrapropylammonium enthaltenden, aber ansonsten erfindungsgemäß hergestellten Titansilikate diese Bande erst nach einer Temperaturbehandlung bei 350 bis 600°C, insbesondere bei Temperaturen zwischen 450 und 550°C, aufweisen. Eine Charakterisierung der Titansilikate ist auch über ihr spezifisches Röntgendiffraktogramm möglich, wobei die im Wesentlichen aminfreien Titansilikate, also solche, die 0 bis 500 ppm, bevorzugt 0,001 bis 200 ppm, besonders bevorzugt 0,01 bis 50 ppm basische (aminhaltige) Komponenten enthalten, ein der orthohombischen MFI-Struktur entsprechendes Diffraktogramm haben.

Die nach dem vorliegenden Verfahren herstellbaren Titansilikatzeolithe können eingesetzt werden zur katalytischen Umwandlung organischer Moleküle. Umsetzungen dieser Art sind beispielsweise Oxidationen, die Epoxidation von Olefinen wie Propylenoxid aus Propylen und H₂O₂, die Hydroxilierung von Aromaten wie Hydrochinon aus Phenol und H₂O₂, die Umwandlung von Alkanen zu Alkoholen, Aldehyden und Säuren, Isomerisierungsreaktionen wie die Umwandlung von Epoxiden zu Aldehyden sowie weitere in der Literatur mit solchen Katalysatoren beschriebenen Reaktionen wie beispielsweise in W. Hölderich, "Zeolites: Catalysts for the synthesis of organic compounds", Elsevier, Stud. Surf. Sci. Catal., Vol. 49, Amsterdam (1989), S. 69 bis 93 und insbesondere für mögliche Oxidationsreaktionen von B. Notari in Stud. Surf. Sci. Catal., Vol. 37 (1987) 413 bis 425 beschrieben.

Die besondere Porenstruktur des Materials und die einfache Herstellung ermöglichen es, daß die erfindungsgemäß herstellbaren Titansilikatzeolithe als mikroporöse Adsorbentien, beispielsweise zur Abtrennung organischer Moleküle oder ihrer Isomeren in flüssiger oder gasförmiger Phase eingesetzt werden können.

Die nachfolgenden Beispiele sollen das erfindungsgemäße Herstellverfahren, sowie die katalytischen Eigenschaften der erhaltenen Titansilikate mit MFI-Struktur, näher erläutern.

### Beispiele

### Beispiel 1

Dieses Vergleichsbeispiel beschreibt die Herstellung eines Titansilikats mit MFI-Struktur, wie es nach dem Stand der Technik in EP-A-376 453 beschrieben ist.

30 ml Tetraethylorthotitanat (Aldrich Chemical Company) wurden unter Rühren (300 U/min) innerhalb von 15 Minuten in 375 ml deionisiertes Wasser getropft, welches zuvor auf 2°C abgekühlt wurde. Danach wurde mit 360 ml kalter Wasserstoffperoxidlösung (30 Gew.-%) versetzt, worauf sich eine orangerote Lösung bildete, die für die Dauer von 2 Stunden gerührt wurde. Danach wurden 625 ml wäßrige Tetrapropylammoniumhydroxidlösung (20-Gew.-%, Fluka) und nach einer weiteren Stunde 100 g kolloidale Silicasol-Lösung (40 Gew.-% SiO₂, Ludox AS-40, Du Pont) zugegeben. Diese Mischung wurde über Nacht bei Raumtemperatur aufbewahrt, am nächsten Tag bei 80°C unter Rühren (300 U/min) für die Dauer von 7 Stunden gekocht, in einen 2 l fassenden Druckrührbehälter eingefüllt und für die Dauer von 240 Stunden bei 175°C zur Reaktion gebracht.

Das erkaltete Reaktionsgemisch wurde abfiltriert, der Filterkuchen mehrmals mit deionisiertem Wasser neutralgewaschen, bei 120°C über Nacht getrocknet und abschlieáend bei 550°C in Luftatmosphäre kalziniert.

Bezogen auf eingesetztes SiO₂ betrug die Ausbeute an Titanzeolith 93 %. Nach dem Röntgenbeugungsmuster handelt es sich um reinen Titanzeolith vom Silikalittyp.

### Beispiel 2

Dieses Synthesebeispiel beschreibt den erfindungsgemäßen Einfluß beim Wechsel von Tetrapropylammoniumhydroxid zu Tetrapropylammoniumbromid unter gleichzeitiger Verwendung von Ammoniaklösung.

In einem mit Rührer und Rückflußkühler versehenen Glaskolben werden 45,1 g deionisiertes Wasser auf 5°C abgekühlt. Dazu tropft man innerhalb von 15 Minuten 6,9 g Tetraisopropylorthotitanat und 81,4 g Wasserstoffperoxidlösung (30 Gew.-%). Zu der entstandenen orangeroten Lösung fügt man 211,0 g einer Ammoniaklösung (25 Gew.-%) und läßt den entstandenen Ansatz sich über Nacht auf Raumtemperatur erwärmen. Abschließend wird für die Dauer von 3 Stunden auf 80°C unter Rühren erwärämt. Eventuell eintretender Gewichtsverlust wird durch Zugabe einer entsprechenden Menge an Ammoniaklösung ausgeglichen.

Von dieser so vorbereiteten Lösung werden 156,8 g mit 56 g Tetrapropylammoniumhydroxidlösung (20 % in Wasser) und 52,8 g Ludox AS-40 Silicasol (Du Pont) innerhalb von 3 Minuten gemischt, in einen teflonausgekleideten Autoklavenbehälter eingefüllt und druckdicht verschlossen. Dies wird im folgenden als Ansatz A bezeichnet.

Weitere 156,5 g der eingangs vorbereiteten Lösung werden mit 14,7 g Tetrapropylammoniumbromid in 44,8 g Wasser und 53,1 g Ludox AS-40 Silicasol (Du Pont) innerhalb von 3 Minuten gemischt, in einen teflonausgekleideten Autoklavenbehälter eingefüllt, und druckdicht verschlossen. Dies wird im folgenden als Ansatz B bezeichnet.

Die Ansätze A und B werden getrennt voneinander bei einer Temperatur von 183 bis 185°C innerhalb von 192 Stunden zur Reaktion gebracht. Die kristallinen Reaktionsprodukte werden abfiltriert, neutralgewaschen, getrocknet und an Luft bei 500°C für die Dauer von 5 Stunden kalziniert.

Die Eigenschaften beider Ansätze sind nachfolgend in Tabelle 2 gegenübergestellt.

**Tabelle 2**

| Ansatz | Ausbeute | Si/Ti molar | Kalium Gew.-% | Größe µm | Form |
|---|---|---|---|---|---|
| A | 96 % | 36 | 0,89 | 5 | globulär |
| B | 95 % | 36 | 0,0012 | 26 | plättchenförmig |

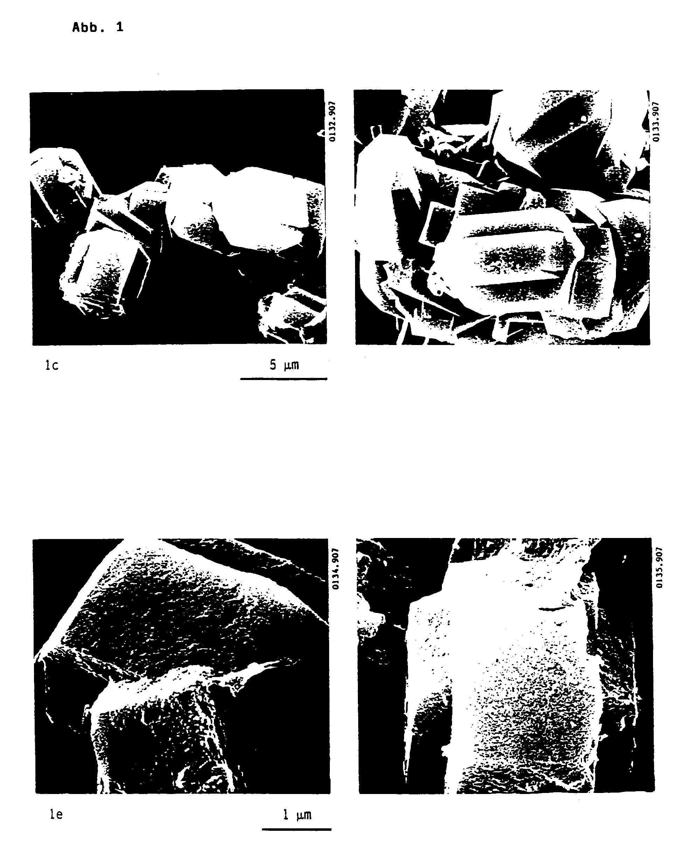

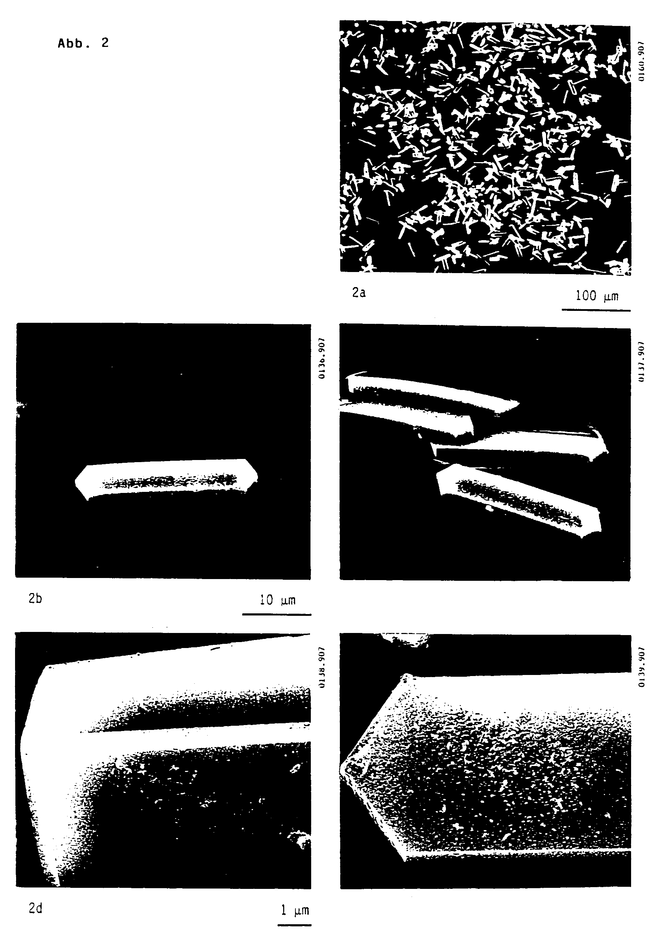

Der unterschiedliche Habitus der Kristalle aus den Ansätzen A und B ist deutlich aus den Abbildungen 1 und 2 zu erkennen. Die Kristallite aus Ansatz A, mit dem teuren Tetrapropylammoniumhydroxid hergestellt, sind verwachsen und von eher globulärer Form, während die Kristalle aus Ansatz B, mit Tetrapropylammoniumbromid kristallisiert, sehr einheitliche Größen haben und sich mit dem länglichen plättchenähnlichen Aussehen deutlich von Ansatz A hinsichtlich Form und Größe unterscheiden.

Die mit Tetrapropylammoniumhydroxid hergestellten Kristalle (Ansatz A) haben einen sehr viel höheren Gehalt an verunreinigendem Kalium verglichen mit den erfindungsgemäß durch geringe Mengen Tetrapropylammoniumbromid synthetisierten Titansilikaten aus Ansatz B.

### Beispiel 3

Beschreibt die erfindungsgemäße Herstellung aus einem vergrößerten gerührten Reaktionsansatz.

In einem mit Rührer und Rückflußkühler versehenen Glaskolben werden 112,5 g deionisiertes Wasser auf 5°C abgekühlt. Dazu tropft man innerhalb von 15 Minuten 34,7 g Tetraisopropylorthotitanat und 203,6 g Wasserstoffperoxidlösung (30 Gew.-%). Zu der entstandenen orangeroten Lösung fügt man 527,5 g einer Ammoniaklösung (25 Gew.-%) und läßt den entstandenen Ansatz sich über Nacht auf Raumtemperatur erwärmen. Abschließend wird für die Dauer von 3 Stunden auf 80°C unter Rühren erwärmt. Eventuell eintretender Gewichtsverlust wird durch Zugabe einer entsprechenden Menge an Ammoniaklösung ausgeglichen. Die so hergestellte Lösung wird mit 73,5 g Tetrapropylammoniumbromid, 224,8 g Wasser und 264,1 g Ludox AS-40 Silicasol in einen Stahlautoklaven mit Rührvorrichtung eingefüllt.

Im Laufe von 168 Stunden bei einer Temperatur von 185°C wird die Reaktionsmischung unter Rühren bei 100 U/min umgesetzt, nach dem Abkühlen das kristalline Produkt abfiltriert, neutralgewaschen, getrocknet und bei 500°C innerhalb 5 Stunden in Luftatmosphäre kalziniert.

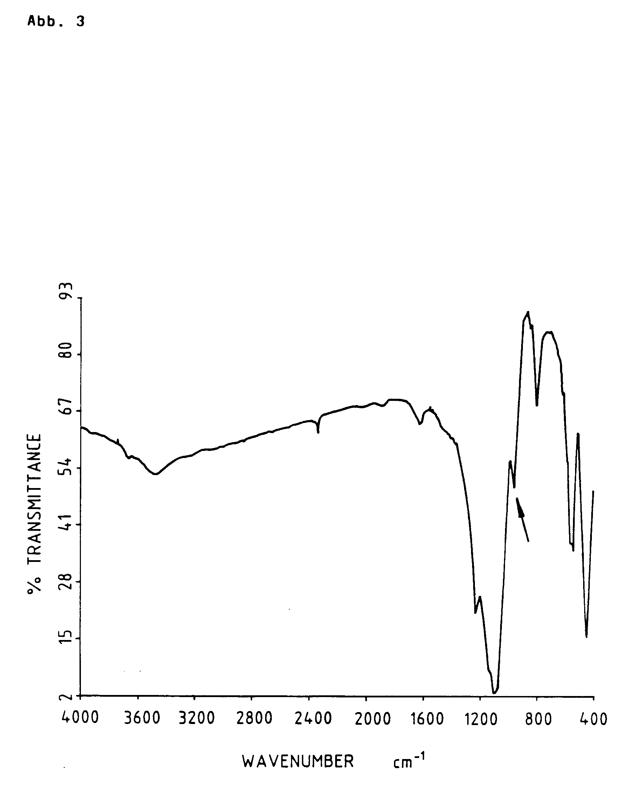

Das Produkt zeigt das typische Röntgendiffraktogramm des TS-1 Titansilikalits. Die Kristalle haben eine Größe von 2 bis zu 25 µm mit einem plättchenförmigem Habitus. Die IR-Aufnahme zeigt deutlich eine scharfe Bande bei 955 cm⁻¹ (vgl. Abb. 3). Die chemische Analyse ergibt ein molares Verhältnis im Produkt von Si/Ti = 16,6. Bezogen auf eingesetztes SiO₂ betrug die Ausbeute an kristallinem, kalzinierten Produkt 96,3 %.

### Beispiel 4

Beschreibt die erfindungsgemäße Herstellung unter Verwendung einer pyrogenen Kieselsäure als Silicaquelle.

In einem mit Rührer und Rückflußkühler versehenen Glaskolben werden 45,2 g deionisiertes Wasser auf 5°C abgekühlt. Dazu tropft man innerhalb von 15 Minuten 6,9 g Tetraisopropylorthotitanat und 81,5 g Wasserstoffperoxidlösung (30 Gew.-%). Zu der entstandenen orangeroten Lösung fügt man 211,0 g einer Ammoniaklösung (25 Gew.-%) und läßt den entstandenen Ansatz sich über Nacht auf Raumtemperatur erwärmen. Abschließend wird für die Dauer von 3 Stunden auf 80°C unter Rühren erwärmt. Eventuell eintretender Gewichtsverlust wird durch Zugabe einer entsprechenden Menge an Ammoniaklösung ausgeglichen. Die so hergestellte Lösung wird mit 14,7 g Tetrapropylammoniumbromid, 75,5 g Wasser und 22,1 g Aerosil-200 (Degussa, pyrogene Kieselsäure) in einen Stahlautoklaven eingefüllt.

Im Laufe von 168 Stunden bei einer Temperatur von 185°C wird die Reaktionsmischung umgesetzt, nach dem Abkühlen das kristalline Produkt abfiltriert, neutralgewaschen, getrocknet und bei 500°C innerhalb 5 Stunden in Luftatmosphäre kalziniert.

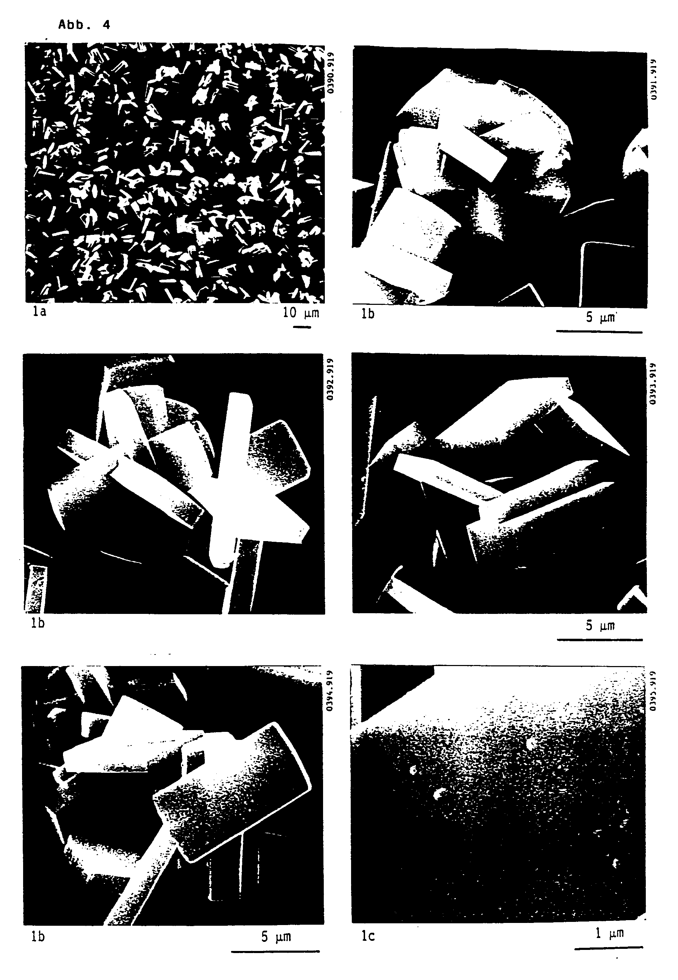

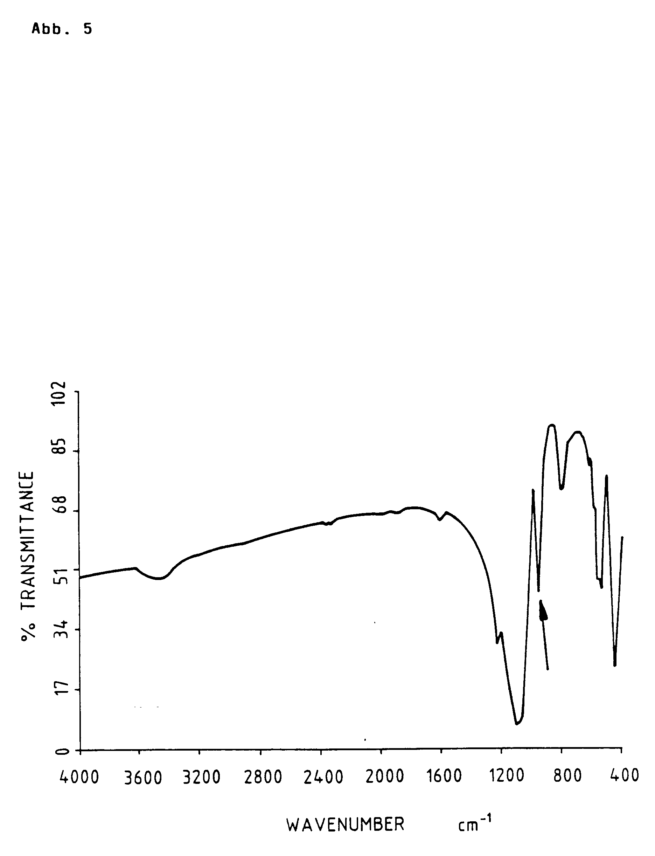

Das Produkt zeigt das typische Röntgendiffraktogramm des TS-1 Titansilikalits. Die Kristalle haben eine gleichförmige Größe von ca. 8 µm mit einem plättchenförmigem Habitus (vgl. Abb. 4). Die IR-Aufnahme der Probe zeigt deutlich eine scharfe Bande bei 960 cm⁻¹ (vgl. Abb. 5). Die chemische Analyse ergibt ein molares Verhältnis im Produkt von Si/Ti = 37,7. Die Verunreinigungen durch Alkali betrugen nur 0,0015 Gew.-% Natrium und 0,0045 Gew.-% an Kalium. Bezogen auf eingesetztes SiO₂ ergab sich eine Ausbeute an kristallinem, kalzinierten Produkt von 90,3 %.

### Beispiel 5

Beschreibt die Verwendung des im Vergleichsbeispiel 1 hergestellten Titanzeoliths zur katalytischen Ammonoximierung von Cyclohexanon zum Cyclohexanonoxim.

In einem Glasdruckbehälter wurden 1,75 g des aus Beispiel 1 hergestellten kalzinierten Titanzeoliths mit 10,5 g Cyclohexanon, 16,0 g wäßriger Ammoniaklösung in 25 g tert.-Butanol als Lösungsmittel unter ständigem Rühren gelöst. Die Temperatur wurde auf 80°C angehoben und innerhalb von 15 Minuten 18,4 ml einer Wasserstoffperoxidlösung (30 Gew.-%) zudosiert. Die einsetzende Reaktion wurde dann noch für die Dauer von 5 Stunden durchgeführt. Der erkaltete Reaktionsaustrag wurde zur Abtrennung der organischen Phase mehrfach mit Toluol versetzt und ausgeschüttelt. Die Analyse der Produkte erfolgte gaschromatographisch.

Es wurde gefunden, daß bei einem Umsatz an Cyclohexanon von 67,8 % mit einer Selektivität von 61,5 % das Zielprodukt Cyclohexanonoxim gebildet wurde.

### Beispiel 6

Beschreibt die Verwendung des im Beispiel 3 hergestellten Titanzeoliths nach dem erfindungsgemäßen Verfahren zur katalytischen Ammonoximierung von Cyclohexanon zum Cyclohexanonoxim.

In einem Glasdruckbehälter wurden 4,2 g des aus Beispiel 3 hergestellten kalzinierten Titanzeoliths mit 10,5 g Cyclohexanon, 23,4 g wäßriger Ammoniaklösung in 23,4 g tert.-Butanol als Lösungsmittel unter ständigem Rühren gelöst. Die Temperatur wurde auf 80°C angehoben und innerhalb von 15 Minuten 18,1 ml einer Wasserstoffperoxidlösung (30 Gew.-%) zudosiert. Die einsetzende Reaktion wurde dann noch für die Dauer von 5 Stunden durchgeführt. Der erkaltete Reaktionsaustrag wurde zur Abtrennung der organischen Phase mehrfach mit Toluol versetzt und ausgeschüttelt. Die Analyse der Produkte erfolgte gaschromatographisch.

Es wurde gefunden, daß bei einem Umsatz an Cyclohexanon von 89,4 % mit einer Selektivität von 94,2 % das Zielprodukt Cyclohexanonoxim gebildet wurde.

### Verzeichnis der Abbildungen

- Abbildung 1:: Titansilikalit-Kristalle aus Ansatz A mit Tetrapropylammoniumhydroxid hergestellt
- Abbildung 2:: Titansilikalit-Kristalle aus Ansatz B mit Tetrapropylammoniumbromid hergestellt
- Abbildung 3:: IR-Spektrum eines erfindungsgemäß hergestellten Titansilkalits aus einem gerührten Syntheseansatz
- Abbildung 4:: REM-Aufnahme von erfindungsgemäß mit pyrogener Kieselsäure hergestelltem Titansilikalit
- Abbildung 5:: IR-Spektrum von erfindungsgemäß mit pyrogener Kieselsäure hergestelltem Titansilikalit

## Patentansprüche

1. Verfahren zur Herstellung von im wesentlichen alkalifreien Titansilikat-Kristallen mit Zeolithstruktur, dadurch gekennzeichnet, daß man eine SiO₂-haltige Mischung aus Wasser, gelöstem Tetra-n-propylammoniumhalogenid mit einem molaren Verhältnis von Tetraalkylammoniumverbindung/SiO₂ im Bereich 0,05:1 bis 0,015:1, einer Titankomponente und Ammoniak in Abwesenheit eines Metallhydroxids oder eines Metallsalzes bei einem molaren Verhältnis von Ammoniak/Tetraalkylammonium von 3:1 bis 200:1 hydrothermal umsetzt.

2. Verfahren nach Anspruch 1, dadurch gekennzeichnet, daß man die hydrothermale Kristallisation unter autogenem Druck bei einer Temperatur von 100°C bis 250°C durchführt.

3. Verfahren nach Anspruch 1, dadurch gekennzeichnet, daß man SiO₂ als wäßrige kolloidale Lösung, in Form einer pyrogenen Kieselsäure, in Form einer hydrolysierbaren Tetraalkoxysilan-Verbindung und/oder in Form vorgefertigter SiO₂-Formkörper mit einem Verhältnis von SiO₂/H₂O von 0,07:1 bis 0,025:1 in der Kristallisationsmischung einsetzt.

4. Verfahren nach Anspruch 1, dadurch gekennzeichnet, daß man als Titankomponente eine lösliche wäßrige oder alkoholische Peroxotitanatlösung in einem Verhältnis von SiO₂/TiO₂ von 10:1 bis 1500:1 verwendet.

5. Aminfreie Titansilikat-Zeolithe, dadurch gekennzeichnet, daß sie nach den Ansprüchen 1 bis 4 hergestellt werden und zusätzlich einer Temperaturbehandlung bei 350°C bis 600°C unterzogen werden.

6. Titansilikate nach dem Verfahren in Anspruch 5, dadurch gekennzeichnet, daß sie ein mit Cu-K(alpha)-Strahlung gemessenes Röntgenbeugungsdiagramm mit mindestens den folgenden Beugungslinien der Tabelle 1 haben:
| Interplanarer Abstand (Angström) | Relative Intensität I/I° * 100 |
|---|---|
| 11.1 - 11.3 | 20 - 85 |
| 9.96 - 10.01 | 50 - 100 |
| 4.99 - 5.01 | 10 - 20 |
| 3.84 - 3.86 | 30 - 50 |
| 3.81 - 3,83 | 70 - 100 |
| 3.74 - 3.77 | 10 - 30 |
| 3.72 - 3.73 | 5 - 30 |
| 3.64 - 3.65 | 5 - 25 |
| 3.32 | 5 - 15 |
| 3.05 | 5 - 10 |
| 2.98 | 5 - 10 |

7. Verwendung der nach Anspruch 1 bis 6 hergestellten Titansilikat-Zeolithe als Katalysatoren zur Umsetzung organischer Moleküle.

8. Verwendung der nach Anspruch 1 bis 6 hergestellten Titansilikat-Zeolithe als Adsorbentien zur Stofftrennung von organischen und anorganischen Molekülen.

9. Verwendung der nach Anspruch 1 bis 6 hergestellten Titansilikat-Zeolithe als binderfreie Katalysatoren in Pulverform zur katalytische Umsetzungen organischer Moleküle in Wirbelschicht-, Rieselbett- oder Suspensionsfahrweise.

## Claims

1. A process for preparing essentially alkali-free titanium silicate crystals having a zeolite structure, which comprises reacting an SiO₂-containing mixture of water, dissolved tetra-n-propylammonium halide in a molar ratio of tetraalkylammonium compound/SiO₂ within the range from 0.05:1 to 0.015:1, a titanium component and ammonia in the absence of a metal hydroxide or salt under hydrothermal conditions in a molar ratio of ammonia/ tetraalkylammonium of from 3:1 to 200:1.

2. A process as claimed in claim 1, wherein the hydrothermal crystallization is carried out at from 100°C to 250°C under autogenous pressure.

3. A process as claimed in claim 1, wherein SiO₂ is added to the crystallization mixture in a ratio of SiO₂/ H₂O of from 0.07:1 to 0.025:1 in the form of an aqueous colloidal solution, in the form of a pyrogenic silica, in the form of a hydrolyzable tetraalkoxysilane compound and/or in the form of prefabricated SiO₂ moldings.

4. A process as claimed in claim 1, wherein the titanium component is a soluble aqueous or alcoholic peroxotitanate solution used in a ratio of SiO₂/TiO₂ of from 10:1 to 1500:1.

5. Amine-free titanium silicate zeolites prepared as claimed in any of the claims 1 to 4 and additionally subjected to a heat treatment at from 350°C to 600°C.

6. Titanium silicates by the process of claim 5, having a Cu-K(alpha) X-ray diffraction diagram comprising at least the following diffraction lines of Table 1:
| Interplanar spacing (angström) | Relative intensity I/I° * 100 |
|---|---|
| 11.1 - 11.3 | 20 - 85 |
| 9.96 - 10.01 | 50 - 100 |
| 4.99 - 5.01 | 10 - 20 |
| 3.84 - 3.86 | 30 - 50 |
| 3.81 - 3.83 | 70 - 100 |
| 3.74 - 3.77 | 10 - 30 |
| 3.72 - 3.73 | 5 - 30 |
| 3.64 - 3.65 | 5 - 25 |
| 3.32 | 5 - 15 |
| 3.05 | 5 - 10 |
| 2.98 | 5 - 10 |

7. The use of the titanium silicate zeolites prepared as claimed in any of claims 1 to 6 as catalysts for reacting organic molecules.

8. The use of the titanium silicate zeolites prepared as claimed in any of claims 1 to 6 as adsorbents for separating organic and inorganic molecules.

9. The use of the titanium silicate zeolites prepared as claimed in any of claims 1 to 6 as binder-free catalysts in powder form for catalytic reactions of organic molecules in fluidized bed, trickle bed or suspension processes.

## Revendications

1. Procédé de préparation de cristaux de silicate de titane essentiellement dépourvus d'alcalis, à structure zéolitique, caractérisé en ce que l'on fait réagir un mélange d'eau, d'halogénure de tétra-n-propylammonium dissous, contenant du SiO₂, avec un rapport molaire composé de tétraalkylammonium/SiO₂ qui varie de 0,05:1 à 0,015:1, un composant du titane et de l'ammoniac, en l'absence d'un hydroxyde de métal ou d'un sel de métal, à un rapport molaire ammoniac/tétraalkylammonium de 3:1 à 200:1, par voie hydrothermique.

2. Procédé suivant la revendication 1, caractérisé en ce que l'on entreprend la cristallisation hydrothermique à une température de 100°C à 250°C sous la pression autogène.

3. Procédé suivant la revendication 1, caractérisé en ce que l'on utilise du SiO₂ sous forme de solution aqueuse colloïdale, sous forme d'un acide silicique pyrogéné, sous forme d'un composé de tétraalcoxysilane hydrolysable et/ou sous forme de corps moulés de SiO₂ apprêtés au préalable, avec un rapport SiO₂/H₂O de 0,07:1 à 0,025:1 dans le mélange de cristallisation.

4. Procédé suivant la revendication 1, caractérisé en ce que l'on utilise, à titre de composant du titane, une solution aqueuse ou alcoolique de peroxotitanate soluble dans un rapport SiO₂/TiO₂ de 10:1 à 1500:1.

5. Zéolites de silicate de titane dépourvus d'amine, caractérisés en ce qu'ils ont été préparés par mise en oeuvre du procédé suivant l'une quelconque des revendications 1 à 4 et supplémentairement soumis à un traitement thermique à une température de 350 à 600°C.

6. Silicates de titane obtenus par mise en oeuvre du procédé suivant la revendication 5, caractérisés en ce qu'ils présentent le diagramme de diffraction des rayons X, mesuré avec le rayonnement Cu-K(alpha) avec au moins les courbes de diffraction suivantes du tableau 1:
| Distance interplanaire (angströms) | Intensité relative I/I° * 100 |
|---|---|
| 11,1 - 11,3 | 20 - 85 |
| 9,96 - 10,01 | 50 - 100 |
| 4,99 - 5,01 | 10 - 20 |
| 3,84 - 3,86 | 30 - 50 |
| 3,81 - 3,83 | 70 - 100 |
| 3,74 - 3,77 | 10 - 30 |
| 3,72 - 3,73 | 5 - 30 |
| 3,64 - 3,65 | 5 - 25 |
| 3,32 | 5 - 15 |
| 3,05 | 5 - 10 |
| 2,98 | 5 - 10 |

7. Utilisation des zéolites de silicate de titane préparés par mise en oeuvre des procédés suivant l'une quelconque des revendications 1 à 6 à titre de catalyseurs pour la conversion de molécules organiques.

8. Utilisation des zéolites de silicate de titane préparées suivant l'une quelconque des revendications 1 à 6, à titre d'adsorbants pour la séparation de substances de molécules organiques et inorganiques.

9. Utilisation des zéolites de silicate de titane préparés suivant l'une quelconque des revendications 1 à 6 à titre de catalyseur dépourvus de liants, sous forme pulvérulente, pour des conversions catalytiques de molécules organiques selon le mode opératoire en couche fluidisée ou turbulente, en lit fluide, ou en suspension.
